Europäisches Patentamt

European Patent Office

Office européen des brevets

(11) Publication number: **0 120 638**
**B1**

(12) **EUROPEAN PATENT SPECIFICATION**

(45) Date of publication of patent specification: **26.04.89**

(21) Application number: **84301595.9**

(22) Date of filing: **09.03.84**

(51) Int. Cl.⁴: **A 61 K 7/08, C 11 D 1/14**

(54) **Concentrated aqueous surfactant.**

(30) Priority: **11.03.83 GB 8306719**

(43) Date of publication of application:
**03.10.84 Bulletin 84/40**

(45) Publication of the grant of the patent:
**26.04.89 Bulletin 89/17**

(84) Designated Contracting States:
**AT BE CH DE FR IT LI NL**

(56) References cited:
**FR-A-2 325 639**
**GB-A-2 022 125**
**US-A-3 821 124**

(73) Proprietor: **Albright & Wilson Limited**
**Albright & Wilson House Hagley Road West**
**Oldbury Warley West Midlands, B68 ONN (GB)**

(72) Inventor: **Akred, Brian John**
**12 Cross Lane**
**Whitehaven Cumbria (GB)**

(74) Representative: **Savidge, Roger Gordon**
**Madgwick et al**
**c/o Albright & Wilson Limited 1 Knightsbridge**
**Green**
**London SW1X 7QD (GB)**

EP 0 120 638 B1

Courier Press, Leamington Spa, England.

## Description

The present invention relates to concentrated aqueous surfactants and in particular to highly concentrated pourable alkyl sulphates having mixed cations.

It has long been known that alkyl sulphates are normally fully dispersible in water to form fluid micellar solutions (the $L_1$ phase) at concentrations up to about 30% Active (i.e. 30% by weight of surface active matter based on the total weight of solution). At higher concentrations phase changes occur leading to the formation of viscous liquid crystal phases. The majority of alkyl sulphates form an essentially immobile liquid crystal phase (the $M_1$ phase) at concentrations substantially above about 35 to 40% Active. At higher concentrations further phase changes occur culminating in the formation of a hydrated solid phase at concentrations typically around 80 to 100% Active.

It will be understood that the concentration at which the foregoing phase changes occur may vary considerably according to the nature of the alkyl groups, and the cations present.

The nature of the phases formed at different concentrations are also profoundly altered by the presence of any substantial amounts of non active material such as electrolytes, solvents and phase modifiers.

Until recently it was assumed that the concentration corresponding to the upper boundary of the $L_1$ phase (typically about 30—35% Active) represented the maximum concentration at which it was practicable to supply binary mixtures of alkyl sulphate and water, since the immobile $M_1$ phase and hydrated solid are difficult and intractable materials to handle, dilute or mix with other ingredients. Higher pourable concentrations could sometimes be obtained by adding substantial quantities of solvents such as ethanol or of phase modifiers such as urea. These greatly increase the cost of the composition, however, and were not always compatible with customers' desired end use formulations.

Accordingly there was generally no alternative but to accept the disadvantages of transporting and storing two parts of water for every part of alkyl sulphate.

In our BP1,488,352 we described how certain alkyl sulphates, namely those of ammonium, amines and mixtures thereof, form a pourable liquid crystal phase (herein referred to as the "G" phase) at ambient temperature and at concentrations above the upper boundary of the $M_1$ phase but below the lower boundary of the hydrated solid phase (typically between about 65% and about 75% active).

In particular in example 4 of the aforesaid British patent there is described a product which is a highly concentrated, pourable mixture of ammonium, monoethanolamine and triethanolamine alkyl sulphates, an analog of which has proved of commercial value as an ingredient of shampoos and cleaning formulations. The viscosity, on dilution of the aforesaid product according to the Patent Example, is undesirably high, requiring the addition of some inorganic salts such as chloride and/or sulphate to provide the commercial product, which meets desired specifications. The commercial product has a setting point of about 24—26°C which is high enough to cause some inconvenience in handling the product in certain circumstances.

We have now discovered a novel composition which generally matches the aforesaid product in performance, but which has a substantially lower setting point, and which, on dilution, generally provides solutions of satisfactory viscosity with substantially reduced need for extraneous additives. The novel product can be handled commercially at significantly higher Active concentrations than the aforesaid product of Example 4 of our British patent or its commercial analog.

Our invention provides a composition consisting essentially of an aqueous solution of a mixed ammonium, monoethanolamine, isopropanolamine alkyl sulphate wherein the alkyl group has an average of from 10 to 18 carbon atoms, said composition having a concentration such that it is a pourable composition consisting, at least predominantly "G" phase at normal ambient temperature.

Typically the total proportion of base used to prepare the product of the invention is sufficient to provide a pH, measured on a 2% solution of the product, of from 4 to 8 preferably 5 to 7, more preferably 5.5 to 6.5 e.g. 5.9 to 6.2.

The proportions of isopropanolamine and of ammonia, measured as concentrated aqueous ammonium hydroxide solution of specific gravity 0.88, are typically each from 2:1 to 4:1 by weight based on the weight of monoethanolamine, preferably 2.5:1 to 3.5:1, e.g. 2.8:1 to 3.2:1.

The product is preferably prepared by neutralising a $C_{12-14}$ alkyl sulphonic acid. Preferably the Active matter in the composition has a mean molecular weight of from 290 to 350, more preferably 300 to 340, typically 310 to 330 e.g. 315 to 325.

The compositions of our inventions will usually contain small amounts of non surface active matter, such as unsulphated fatty alcohols, which are normally present in commercial alkyl sulphates. We prefer however that the total proportion of non-active non-ionic organic matter be less 10% of the weight of the composition more preferably less than 5% e.g. less than 3.5%. Inorganic materials, and especially inorganic salts such alkali metal and/or, preferably ammonium sulphate or chloride, may be added to the composition in minor amounts, for example to adjust the viscosity of solution formed on dilution. In general we prefer that the total non-surfactant salt content of the composition should not exceed 10% and should preferably be less than 5% by weight, e.g. less than 3%.

In general non-active material is not added to compositions according to our invention in amounts sufficient to suppress altogether the formation, at lower active concentrations of an immobile $M_1$ phase. We have found that products with particularly satisfactory properties can be prepared by including 1.2 to 2.8% by weight of sulphate and less than 0.1% chloride, without any added organic solvent or phase modifier, other than the residual, unsulphated fatty alcohol in amounts below 3.5% by weight.

Compositions according to our invention may conveniently be adjusted by additions of inorganic salt to provide a viscosity of 35±15 cS at 20°C, on dilution to 16% Active concentration.

The Active concentration of the compositions of our invention will depend upon the concentration corresponding to the $M_1$/G phase transition and the concentration of the G phase/hydrated solid phase transition, the composition having a concentration above the former phase transition and below the latter. Preferably the composition has a concentration which corresponds substantially to the viscosity minimum which lies between the aforesaid phase transition concentrations, preferably within ±10% of the minimum more preferably ±5% e.g. ±2%.

The actual concentrations at which the viscosity minimum and the two phase transitions fall, will vary according to particular alkyl sulphonic acid, the proportions of the bases and the amount of non-active matter. Typically the $M_1$/G phase transition occurs above 45%, usually above 50% active concentration, between 60 and 70% by weight active concentration. The G phase/hydrated solid transition usually occurs above 60% e.g. between 65 and 85% Active concentration. The presence of inorganic electrolytes tends to lower both the maximum and minimum concentrations at which a G phase is formed, the latter being lowered by a greater amount that the former, giving a wider range of concentrations which form the G phase and a narrower range which form the $M_1$ phase.

We have found that with compositions containing monoethanolamine isopropanolamine and 0.880 s.g. ammonia in proportions of about 1:3:3, by weight, having a mean molar weight of 320 and containing 1.2% sulphate the $M_1$/G transition occurs at 65% Active and the G/hydrated solid transition at 80% Active concentration. At 2.8% sulphate the $M_1$/G transition occurs at 59% Active.

The concentrations may readily be determined for any particular sample by preparing a product at say 70% Active concentration and observing the sample through a polarising microscope. Characteristic textures are observed according to the phase present. These may be identified by comparison, for example with the examples in the paper by Rosevear, JAOCS Vol. 31, P628 (1954) or with J. Colloid and Interfacial Science, Vol. 30, No. 4, P500. Water may be added to, or evaporated from the sample until the desired phase transition is observed.

Preferably compositions according to our invention are manufactured by neutralising the appropriate alkyl sulphonic acid with an aqueous mixture of bases containing the appropriate amount of water to form the product at the desired G phase concentration. We do not however exclude sequential neutralisation.

Any additives which are required may be added subsequently or, preferably, may be included in the initial reaction mixture.

The invention is illustrated by the following example:—

Example

The example was prepared using a loop neutraliser as described in our aforesaid British patent. A sulphated mixture of dodecyl and tetradecyl normal primary alcohols was used as the acid feed while the base feed comprised a mixture of 9.3 g monoethanolamine, 28.9 g isopropanolamine and 26.1 g concentrated ammonia solution (S.G. 0.880) in 61.7 g water. The temperature was maintained at 35°C and the product manufacture rate was 12 g/min. The pourable G phase product contained 75.6% of active ingredient having a mean molar weight of 320, 2.7% of free fatty matter and 0.9% of sulphate as $SO_4^-$ ion. To this material was added sufficient aqueous ammonium sulphate to affect an adjustment in analysis to 72.0% active ingredient and 1.9% sulphate as $SO_4^-$ ion. This final product was a pourable G phase having a setting point of about 12°C, Dilution viscosity was 29 $mm^2$/s at 20°C measured on a 16% Active solution.

## Claims

1. A concentrated aqueous alkyl sulphate surfactant having a $C_{10-18}$ alkyl group and having a concentration such that it is present as G phase at normal ambient temperatures characterised in that, the cations comprise a mixture of ammonium, monoethanolammonium and isopropanolammonium.

2. A composition according to claim 1 having a pH, when diluted with water to 2% solids, of from 4 to 8.

3. A composition according to claim 2 having a pH, when diluted with water to 2% solids, of from 5.5 to 6.5.

4. A composition according to any foregoing claim wherein the proportion of isopropanolamine is from 2:1 to 4:1 based on the weight of monoethanolamine.

5. A composition according to any foregoing claim wherein the proportion of ammonia, measured as concentrated aqueous ammonium hydroxide solution of specific gravity 0.88 is from 2:1 to 4:1 based on the weight of monoethanolamine.

6. A composition according to any foregoing claim wherein the alkyl groups of the alkyl sulphate anions have an average of from 12 to 14 carbon atoms.

7. A composition according to any foregoing claim containing less than 5% by weight of non surface active, non-ionic organic matter and less than 5% by weight of non-surface active salts.

8. A composition consisting essentially of water, and a mixed ammonium, monoethanolamine, isopropanolamine alkyl sulphate; having a pH of from 5 to 7, when diluted to a concentration of 2% by weight solids; said alkyl group having an average of from 10 to 14 carbon atoms; said ammonia, measured as concentrated aqueous ammonium hydroxide of 0.88 specific gravity, and said isopropanolamine, each being present in a proportion of from 2.5:1 to 3.5:1, based on the weight of monoethanolamine; said composition containing less than 5% each of non-surfactant, non-ionic organic matter and non-surfactant salts, and having a concentration greater than that corresponding to the $M_1$ phase/ G phase transition, but below that corresponding to the G phase/hydrated solid transition.

9. A method for the preparation of a composition according to any foregoing claim which comprises reacting an alkyl sulphuric acid simultaneously, or sequentially in any order, with aqueous ammonia, isopropanolamine and monoethanolamine in the presence of sufficient water to maintain the product in the G phase.

## Patentansprüche

1. Konzentriertes wässeriges Alkylsulfat-Surfactant mit einer $C_{10-18}$-Alkylgruppe und einer solchen Konzentration, daß es bei normalen Raumtemperaturen als G-Phase vorliegt, dadurch gekennzeichnet, daß die Kationen eine Mischung von Ammonium, Monoethanolammonium und Isopropanolammonium enthalten.

2. Zusammensetzung nach Anspruch 1 mit einem pH-Wert von 4 bis 8 bei Verdünnung mit Wasser auf 2% Feststoffe.

3. Zusammensetzung nach Anspruch 2 mit einem pH-Wert von 5,5 bis 6,5 bei Verdünnung mit Wasser auf 2% Feststoffe.

4. Zusammensetzung nach irgendeinem der vorhergehenden Ansprüche, in welcher der Anteil Isopropanolamin von 2:1 bis 4:1, bezogen auf das Gewicht des Monoethanolamins, beträgt.

5. Zusammensetzung nach irgendeinem der vorhergehenden Ansprüche, in welcher der Anteil an Ammoniak, gemessen als konzentrierte wässerige Ammoniumhydroxidlösung mit spezifischem Gewicht 0,88 2:1 bis 4:1, bezogen auf das Gewicht des Monoethanolamins, beträgt.

6. Zusammensetzung nach irgendeinem der vorhergehenden Ansprüche, in welcher die Alkylgruppen der Alkylsulfatanionen im Mittel 12 bis 14 Kohlenstoffatome enthalten.

7. Zusammensetzung nach irgendeinem der vorhergehenden Ansprüche, die weniger als 5 Gew.-% nicht oberflächenaktiver, nichtionischer organischer Substanz und weniger als 5 Gew.-% nicht oberflächenaktiver Salze enthält.

8. Zusammensetzung, die im wesentlichen aus Wasser und einem gemischten Ammonium-, Monoethanolamin-, Isopropanolamin-Alkylsulfat besteht; einen pH-Wert von 5 bis 7 bei Verdünnung auf eine Konzentration von 2 Gew.-% Feststoff aufweist; wobei die genannte Alkylgruppe im Mittel 10 bis 14 Kohlenstoffatome enthält; der Ammoniak, gemessen als konzentriertes wässeriges Ammoniumhydroxid mit einem spezifischen Gewicht von 0,88, und das Isopropanolamin jeweils in einer Menge von 2,5:1 bis 3,5:1 bezogen auf das Gewicht des Monoethanolamins vorliegen; die Zusammensetzung weniger als 5% von jeweils nicht oberflächenaktiver Substanz, nichtionischer organischer Substanz und nicht oberflächenaktiven Salzen enthält und eine Konzentration aufweist, die größer als die ist, die dem Übergang von $M_1$-Phase/G-Phase entspricht, jedoch unterhalb jener liegt, die dem Übergang von G-Phase/hydriertem Feststoff entspricht.

9. Verfahren zur Herstellung einer Zusammensetzung nach irgendeinem der vorhergehenden Ansprüche, bei welchem eine Alkylschwefelsäure gleichzeitig oder in beliebiger Reihenfolge nacheinander mit wässerigem Ammoniak, Isopropanolamin und Monoethanolamin in Gegenwart von ausreichendem Wasser, um das Produkt in der G-Phase zu halten, zur Reaktion gebracht wird.

## Revendications

1. Alkyl sulfate tensio-actif aqueux concentré, comportant un groupe alkyle en $C_{10}$ à $C_{18}$ et ayant une concentration telle que le tensio-actif est présent sous forme d'une phase G aux températures ambiantes normales, produit caractérisé en ce que les cations comprennent un mélange des ions ammonium, monoéthanolammonium et isopropanolammonium.

2. Composition selon la revendication 1, ayant, lors de la dilution avec de l'eau jusqu'à 2% de solides ou d'extrait sec, un pH compris entre 4 et 8.

3. Composition selon la revendication 2, ayant, en cas de dilution avec de l'eau jusqu'à une teneur de 2% en solides ou en extrait sec, un pH de 5,5 à 6,5.

4. Composition selon l'une quelconque des revendications précédentes, dans laquelle la proportion de l'isopropanolamine va de 2:1 à 4:1, sur la base du poids de la monoéthanolamine.

5. Composition selon l'une quelconque des revendications précédentes, dans laquelle la proportion d'ammoniaque, mesurée sous forme d'une solution aqueuse concentrée d'hydroxyde d'ammonium, de densité 0,88, est de 2:1 à 4:1, sur la base du poids de la monoéthanolamine.

6. Composition selon l'une quelconque des revendications précédentes, dans laquelle les groupes alkyles des anions alkyl sulfates comportent en moyenne de 12 à 14 atomes de carbone.

7. Composition selon l'une quelconque des revendications précédentes, contenant moins de 5% en poids de substance non tensio-active, de matière organique non ionique et moins de 5% en poids de sels non tensio-actifs.

8. Composition consistant essentiellement en de l'eau et en un alkyl sulfate mixte d'ammonium, de monoéthanolamine et d'isopropanolamine, ayant un pH de 5 à 7, lorsqu'il y a dilution jusqu'à une concentration de 2% en poids des solides ou de l'extrait sec; ledit groupe alkyle ayant en moyenne de 10 à 14 atomes de carbone; ladite ammoniaque, mesurée sous forme d'un hydroxyde d'ammonium aqueux concentré de densité 0,88, et ladite isopropanolamine, étant chacune présentes en une proportion de 2,5:1 à 3,5:1, sur la base du poids de la monoéthanolamine; ladite composition contenant moins de 5% de substance non tensio-active, moins de 5% de matière organique non ionique et moins de 5% de sels non tensio-actifs, et ayant une concentration supérieure à celle correspondant à la transition phase $M_1$/phase G, mais inférieure à celle correspondant à la transition phase G/solide hydraté.

9. Procédé pour la préparation d'une composition selon l'une quelconque des revendications précédentes, qui comprend la réaction d'un acide alkyl sulfurique simultanément, ou successivement dans un ordre quelconque, avec de l'ammoniaque aqueuse, de l'isopropanolamine et de la monoéthanolamine, en présence d'une quantité d'eau suffisante pour maintenir le produit en phase G.